# EUROPEAN PATENT APPLICATION

(11) **EP 3 831 909 A1**
(43) Date of publication of application: **09.06.2021**
(21) Application number: 19306564.6
(22) Date of filing: 03.12.2019
(51) Int. Cl.: C09K 11/02, G01N 33/53, A61K 49/00, C09B 67/02

(54) **LUMINESCENT ZWITTERIONIC POLYMERIC NANOPARTICLES**

(71) Applicant: Université de Strasbourg, 67000 Strasbourg (FR); Centre National de la Recherche Scientifique, 75016 Paris (FR)
(72) Inventor: KLYMCHENKO, Andrii, 67400 ILLKIRCH (FR); REISCH, Andreas, 68000 COLMAR (FR); RUNSER, Anne, 67100 STRASBOURG (FR); DUJARDIN, Denis, 67140 BARR (FR)
(74) Representative: IPAZ

(57) **Abstract**

The present invention concerns zwitterionic luminescent polymeric nanoparticles, their method of preparation and the use of these nanoparticles in the medical field and in the biological research field.

## Description

The present invention concerns luminescent zwitterionic polymeric nanoparticles, their method of preparation and the use of these nanoparticles in the medical field and in the biological research field.

The emergence of single-molecule and super-resolution fluorescence microscopy have been opening a new area of imaging with the promise to visualize biological processes at a molecular level in living cells. For tracking or visualizing single biomolecules in the cytoplasm, the fluorescent emitters need to be very bright. Indeed, the spatio-temporal resolution is directly linked to the number of collected photons. Recently, dye-loaded polymer nanoparticles have achieved very high brightness through the encapsulation of a large amount of fluorescent dye in nanoparticles (Reisch, A.; Klymchenko, A. S. Fluorescent Polymer Nanoparticles Based on Dyes: Seeking Brighter Tools for Bioimaging. Small 2016, 12 (15), 1968-1992.). Several new strategies, such as fluorescent organic dots with aggregation-induced emission¹ or use of salts of fluorophores with hydrophobic bulky counterions², had been used to overcome aggregation-caused quenching of the fluorophores encapsulated at very high concentrations.

However, in order to be used in biological environments, single-molecule imaging requires not only high brightness of nanoparticles, but also small probe sizes and an absence of non-specific interactions with biological environments.³ Firstly, in order to increase localization precision, the particle diameter needs to be of the order of the size of the biomolecule (e.g. protein) it is intended to label. Secondly, the size and the surface coating of nanoparticles play a crucial role in defining their intracellular behavior and their influence on the behavior of the labeled biomolecules should be limited.^{4,5} Diffusion of nanoparticles in biological environments, such as in cells, can be limited due to structural restrictions and macromolecular crowding effects inside cells.⁶ Etoc. *et al.* determined that particles of ≤50 nm can have Brownian diffusion in the cytosol.⁴ The inventors also showed in the past that the limit core size, for which the NPs can reach almost all parts of the cytosol, is around 23 nm.⁵ At the same time, the surface chemistry also has a major effect on the behavior of nanoparticles in biological systems⁷ and can affect the diffusion and the motion of NPs in the cytosol.⁴ One reason for this is that the surface chemistry will determine interactions with proteins. In particular, the adsorption of proteins leading to the formation of a protein corona affects all aspects of NP behavior from the cellular uptake to their degradation,⁸ and as long as there are strong interactions between proteins and the NP surface, nanoparticles are not able to freely move in the cytosol.⁴ Therefore, the control of the surface properties to reduce (suppress) protein adsorption is essential.

Till now, introducing polyethylene glycol (PEG) is the best known strategy for suppressing protein adsorption on nanoparticles.⁹ However, PEGylation is not fully adapted to produce nanoparticles having a size similar to that of biomolecules (e.g. protein), since the PEG chains needed for efficiently suppressing interactions with proteins also significantly increase the particle diameter by several nanometers and often by around 10 nm.^{10,11}

For example, WO 2018/044688 described a water-dispersible fluorescent particle comprising a mixture of one or more hydrophobic polymers, which are encapsulated in one or more amphiphilic polymers. Said amphiphilic polymers are **block** copolymers comprising at least one hydrophobic segment and at least one hydrophilic segment, such as PS-b-PEG.

An alternative is the use of zwitterionic (ZI) groups, mimicking the outer surface of the cell membrane.¹² The mechanism of suppression of protein adsorption in the case of zwitterions is linked to their very hydrophilic nature and the fact that they contain their own counterion,^{11,12} rather than on steric repulsion as in the case of PEG. Therefore, unlike PEG coatings, zwitterionic coatings can be very thin, limiting the increase of particle diameter. Several approaches have been developed to implement zwitterionic, especially carboxybetaine, sulfobetaine and phosphorylcholine, groups on NP surfaces. However, most of zwitterionic nanoparticles developed in the past are inorganic nanoparticles as quantum dots (QDs),¹³ silica NPS,¹¹ or Au NPs.¹⁴

Till now, in the case of polymeric nanoparticles, zwitterionic polymeric particles are mainly obtained from **block** copolymers bearing a zwitterionic block and a hydrophobic block.

The shortcoming of this approach is that the diameters of these NPs are not satisfactory, which limits their use in *in vivo* environments. By the way, their synthesis process is often very complex.

Thus, in order to improve *in vitro* or *in vivo* detection or tracking of a target biological molecule, it is necessary to provide new families of zwitterionic polymeric nanoparticles having high brightness and controllable smaller size and limited protein surface interactions.

In order to satisfy these requirements, the Inventors of the present invention developed novel luminescent zwitterionic nanoparticles, based on specially designed random hydrophobic polymers, bearing apolar, charged, zwitterionic and reactive groups for bioconjugation.

Contrary to block zwitterionic copolymers known in prior art wherein the zwitterionic block and the hydrophobic block are separated and localized on two ends of the polymer chain, the zwitterionic copolymers forming the nanoparticles of the present invention have both negatively charged groups and zwitterionic groups which are randomly arranged on the polymeric backbone chain among the hydrophobic pendant groups of the copolymer.

Against all odds, it is surprising to observe that this random arrangement of a small amount of zwitterionic groups and negatively charged groups can on the same time further reduce nanoparticle's size and efficiently prevent protein adsorption on nanoparticle's surface while maintaining a high luminescence brightness.

The nanoparticles of the present invention can have a diameter as small as 7-20 nm, that is particularly suitable for being monitored directly at the single particle level in the cytoplasm of living and fixed cells and tissues.

The first aspect of the present invention is to provide a zwitterionic luminescent polymeric nanoparticle, said nanoparticle comprising at least one random copolymer, said random copolymer comprising:
- from 0.5 to 20 mol% of repeating units having a pendant group which is a negatively charged group chosen from phosphonate, sulfonate and carboxylate,
- from 3 to 30 mol%, in particular from 5 to 20 mol%, more particularly from 7 to 17 mol%, of repeating units having a pendant group which is a zwitterionic group,
- from 0 to 5 mol% of repeating units having a pendant group which is a reactive group which is suitable to be functionalized by a natural or synthetic biologically interesting molecule,
- from 60 to 95 mol% of repeating units having a pendant group, which is a hydrophobic group.

Without being bound by any theory, the nanoparticles of the invention probably have a hydrophobic core made up predominantly of the hydrophobic part of the copolymers and of the dye salt. The excellent resistance to protein adsorption and the high mobility of the particles in the cytosol, on the other hand, suggest a very hydrophilic surface with a high density of zwitterionic groups. Thus, the nanoparticles of the invention are supposed to have a core-shell structure with the hydrophobic parts of the amphiphilic polymers predominantly concentrated in the core, responsible for encapsulation of luminescent dyes, and the charged and zwitterionic parts abundant in the shell, responsible for controlling the interactions.

For a given particle-core size, the nanoparticles of the invention make it possible to reduce by a factor of 2 the total size of nanoparticles with respect to that of PEGylated nanoparticles based on the same major monomer. This is due to the smaller size of the particle shell in the case of zwitterionic particles compared to their PEGylated counterparts. The brightness of the particles depends on the number of dyes encapsulated in their core and hence, for a given total size, on the ratio of core to the total particle diameter (core and shell). In consequence, for the same total particle diameter, the brightness of the particles of invention, which have nearly 2-fold larger core diameter because of very thin shell, can be increased by nearly 8-fold. High loading (>20 wt%) and quantum yields of the present particles (>30%) have been confirmed. That means the nanoparticles of the invention are ultra-bright compared to non-zwitterionic nanoparticles.

Thus, the nanoparticles of the present invention combined, on the one hand, efficient dye encapsulation and high quantum yields, resulting in high particle brightness, and on the other hand, excellent stability and resistance to protein adsorption.

As used herein, the term "random copolymer" is meant to a copolymer having two or more kinds of monomers which are polymerized into no particular sequence. In a random copolymer, the individual repeating units are randomly distributed along the backbone chain of the copolymer.

The term "repeating unit" is meant to a unit whose repetition would produce the complete polymer chain by linking the repeating units together successively along the chain.

As used herein, the term "pendant group" with respect to a polymer, is meant to a group of molecules attached to the backbone chain of a polymer.

As a consequence, the pendant group of a major monomer is also the major pendant group of the random copolymer polymerized with that major monomer.

The term "backbone" of polymer is referred to a linear chain of a polymer on which all other long chains or short or both, can be considered as pending.

According to the invention, said random copolymer can be a (C1-C6)alkyl methacrylate based polymer, an (C1-C6)alkyl acrylate based polymer, an acrylamide based polymer, a polyester based polymer, a polyamide (polypetide) based polymer, a styrene based polymer and copolymers thereof.

The term "(C1-C6) alkyl methacrylate based polymer" in the context of the present invention is to be understood as a copolymer which is formed by (C1-C6) alkyl methacrylate as major monomer and other minor monomers.

In the context of the present invention, the terms "(C1-C6)alkyl acrylate based polymer", "acrylamide based polymer", "polyester based polymer", "polyamide based polymer", "styrene based polymer", should be interpreted in the similar way.

Examples of (C1-C6) alkyl methacrylate based polymer can be methyl methacrylate based copolymer, ethyl methacrylate based copolymer, propyl methacrylate based copolymer, isopropyl methacrylate based copolymer, or butyl methacrylate based copolymer.

Examples of polyester based polymer can be cited as, but not limited to, polyglycolic acid (PGA) based copolymer, poly(lactide co-glycolide) (PLGA) based copolymer, Polylactic acid (PLA) based copolymer, or polycaprolactone (PCL) based copolymer.

An embodiment of random copolymer comprised in the nanoparticles of the present invention is represented by the formula I below: 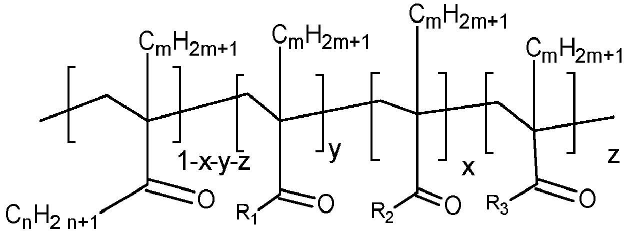 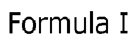 Wherein:
- m is 0 or 1,
- n is an integer chosen from 1 to 6
- R₁ is a zwitterionic group,
- R₂ is a negatively charged group,
- R₃ is a reactive group which is suitable to be functionalized by a natural or synthetic biologically interesting molecule,
- x is in the range from 0.5 to 20 mol%,
- y is in the range from 3 to 30 mol%, in particular from 5 to 20 mol%, more particularly from 7 to 17 mol%,
- z is in the range from 0 to 5 mol%.

A more particular embodiment of a random copolymer comprised in the nanoparticles of the present invention can be represented by the formula Ia below: 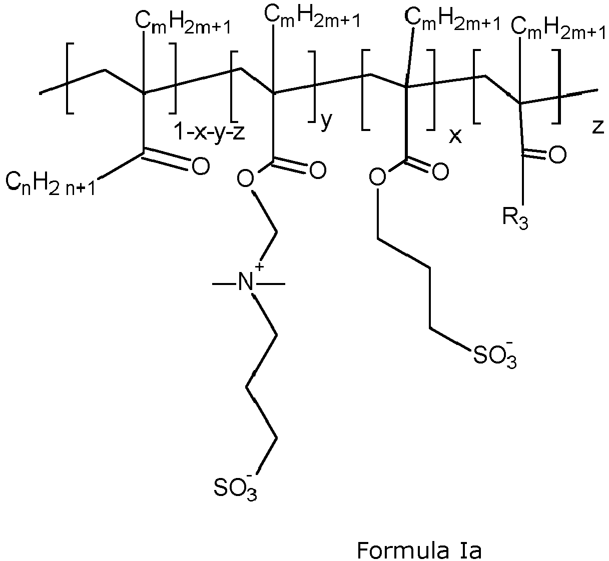 Wherein:
- m is 0 or 1,
- n is an integer chosen from 1 to 6
- x is in the range from 0.5 to 20 mol%,
- y is in the range from 3 to 30 mol%, in particular from 5 to 20 mol%, more particularly from 7 to 17 mol%,
- z is in the range from 0 to 5 mol%,
- R₃ is a reactive group which is suitable to be functionalized by a natural or synthetic biologically interesting molecule.

Another embodiment of a random copolymer comprised in the nanoparticles of the present invention is represented by the formula II below: 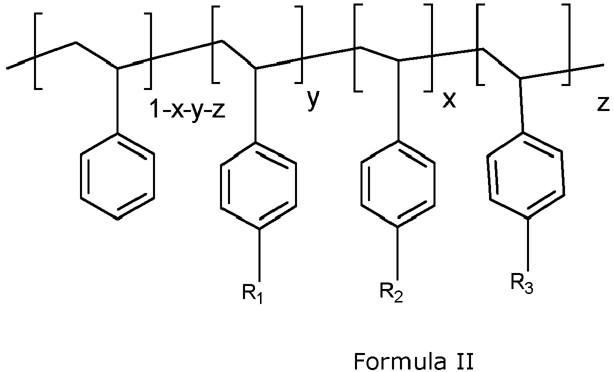 Wherein:
- R₁ is a zwitterionic group,
- R₂ is a negative charged group,
- R₃ is a reactive group which is suitable to be functionalized by a natural or synthetic biologically interesting molecule,
- x is in the range from 0.5 to 20 mol%,
- y is in the range from 3 to 30 mol%, in particular from 5 to 20 mol%, more particularly from 7 to 17 mol%,
- z is in the range from 0 to 5 mol%.

According to a particular embodiment, the zwitterionic luminescent polymeric nanoparticle of the invention comprises a random copolymer that is a (C1-C6)alkyl methacrylate based polymer, said random copolymer comprising :
- from 0.5 to 20 mol% of repeating units having a pendant group which is a negatively charged group chosen from phosphonate, sulfonate and carboxylate,
- from 3 to 30 mol%, in particular from 5 to 20 mol%, more in particular from 7 to 17mol%, of repeating units having a pendant group which is a zwitterionic group,
- from 0 to 5 mol% of repeating units having a pendant group which is a reactive group which is suitable to be functionalized by a natural or synthetic biologically interesting molecule,
- from 70 to 95 mol% of repeating units having a pendant group which is a (C1-C6)alkyl.

One kind of minor pendant groups comprised in the random polymer of the present invention is negative charged groups chosen from phosphonate, sulfonate and carboxylate.

A random copolymer comprised in the nanoparticles of the present invention has from 0.5 to 20% of repeating units having a negatively charged group as pendant group.

Another kind of minor pendant groups comprised in the random polymer of the present invention is zwitterionic group.

The term of "zwitterionic group" is referred to a chemical group which contains at the same time a part having a positive charge and another nonadjacent part having a negative charge. The examples of zwitterionic groups which can be comprised in above described random copolymer can be chosen from ammoniophosphates, ammoniophosphonates, ammoniophosphinates, ammoniosulfonates, ammoniosulfates, ammoniocarboxylates, ammoniosulfonamides, ammoni-sulfon-imides, guanidiniocarboxylates, pyridiniocarboxylates, pyridiniosulfonates, ammonio(alkoxy)dicyanoethenolates, ammonioboronates, sulfoniocarboxylates, phophoniosulfonates, phosphoniocarboxylates.

A random copolymer comprised in the nanoparticles of the present invention has from 3 to 30%, in particular from 5 to 20%, more particularly from 7 to 17%, of repeating units having a zwitterionic group as pendant group.

The presence of very hydrophilic zwitterionic groups in said random copolymer increases hydrophilicity of copolymer. However, the random copolymer comprised in the nanoparticle of the present invention remains basically hydrophobic due to the major part of hydrophobic pendant groups.

Thanks to the presence of zwitterionic groups and negatively charged groups, the nanoparticle of the invention has a particular reduced size.

The diameter of the nanoparticle of the present invention is varied from 5 nm to 200 nm, particularly from 5 nm to 150 nm, more particularly from 5 nm to 100 nm, still more particularly from 5 nm to 50 nm, still more particularly from 7 to 30 nm, still more particularly from 7 to 20 nm.

Nanoparticle's diameter and size distribution can be measured according to a conventional method by electron microscopy or dynamic light scattering.

The nanoparticles of the invention comprise also luminescent dyes.

Within the scope of the present invention, said luminescent dyes can be fluorescent dyes or phosphorescent dyes.

Said luminescent dyes can also be luminescent metal complexes.

In a preferred embodiment, said luminescent dye is a fluorescent dye optionally with its counter-ion, said dye and said counter-ion being encapsulated inside the nanoparticle.

Examples of fluorescent dyes can be cited as, but not limited to, rhodamine derivatives, cyanine derivative, fluorescein derivatives, BODIPY derivatives, aza-BODIPY derivatives, coumarines, squaraines, porphyrins or phthalocyanines.

Their suitable counter-ions for ionic dyes can be an inorganic counter-ion or a bulky organic counter-ion.

Examples of inorganic counterion may include, without limitation, chloride, perchlorate, sulfonate, nitrate, tetrafluoroborate, hexafluorophosphate.

The term "bulky organic counterion" as used herein means a large organic anion bearing aromatic and/or aliphatic residues. Examples of bulky organic counterion can be cited, but not limited to tetrakis(pentafluorophenyl)borate, tetrakis(4-fluorophenyl)borate, tetrakis[3,5 - bis(trifluoromethyl)phenyl]borate, tetrakis[3,5 - bis(1,1,1,3,3,3 - hexafluoro - 2 - methoxy - 2 - propyl)phenyl]borate, tetrakis[perfluorotert-butoxy]aluminate, or tetraphenylborate.

According to some embodiments, the random copolymer of the present invention can also comprise a third kind of minor pendant group that is a reactive group which is suitable to be functionalized by a natural or synthetic biologically interesting molecule.

For example, said reactive group can be a reactive group suitable for "click chemistry", such as azide, tetrazine, alkenyl groups, or other reactive groups which can carry out a cycloaddition, or reactive groups, such as maleimide groups, or active esters, such as pentafluoro phenyl esters or NHS-esters.

This third kind of minor pendant groups makes it possible to combine a natural or synthetic biologically interesting molecule with the luminescent zwitterionic nanoparticles of the invention.

Examples of said natural or synthetic biologically interesting molecule can be, but not limited to, a molecule that recognizes a target biomolecule in cells, tissues or biological fluids, such as an antibody, a fragment of antibody, a ligand, an agonist or antagonist of a natural biological molecule, a peptide, an aptamer, an oligonucleotide, a toxin or a chemical drug.

This kind of luminescent zwitterionic nanoparticles bearing reactive groups is particularly interesting, since they can be easily further functionalized according to destined applications of the nanoparticles.

According to some embodiments, the luminescent zwitterionic polymeric nanoparticle of the present invention can comprise a random copolymer as described before as the only polymeric material.

According to some other embodiments, the luminescent zwitterionic polymeric nanoparticles of the present invention can comprise a random copolymer as described above with another polymer, or comprise at least two kinds of random copolymers as described above.

In a preferred embodiment, the luminescent zwitterionic polymeric nanoparticles of the present invention comprise:
- a first random copolymer as described above with reactive group suitable for nanoparticles functionalization with a biologically interesting molecule, and
- a second random copolymer as described above but free of reactive groups for nanoparticle functionalization.

The mixture of a reactive group-bearing random copolymer with another random copolymer free of reactive group makes it possible to control with more accuracy the quantity of biologically interesting molecules that will be bound to a nanoparticle via these reactive groups, and that will further impact for example the detection sensibility of the nanoparticles, target molecule quantification, or single-molecule tracking.

In a particular embodiment, the zwitterionic polymeric nanoparticle of the invention comprises a first random copolymer and a second random copolymer, the first random copolymer comprising:
- from 0.5 to 20 mol% of repeating units having a pendant group which is negatively charged group chosen from phosphonate, sulfonate and carboxylate,
- from 3 to 30 mol%, in particular from 5 to 20 mol%, more particularly from 7 to 17 mol% of repeating units having a pendant group which is zwitterionic group,
- higher than 0 but lower or equal to 5 mol%, of repeating units having a pendant group which are reactive groups which are suitable to be functionalized by a natural or synthetic biologically interesting molecule,
the second random copolymer being free of reactive groups which are suitable to be functionalized by a natural or synthetic biologically interesting molecule and comprising :
- from 0.5 to 20 mol% of repeating units having a pendant group which is negatively charged group chosen from phosphonate, sulfonate and carboxylate,
- from 3 to 30 mol%, in particular from 5 to 20 mol%, more particularly from 7 to 17 mol%, of repeating units having a pendant group which is zwitterionic group.

In another particular embodiment, the luminescent zwitterionic polymeric nanoparticles of the present invention comprise:
- a first random copolymer as described above with a reactive group suitable for nanoparticles functionalization with a first type of biologically interesting molecule, and
- a second random copolymer as described above with a reactive group suitable for nanoparticle functionalization with a second type of biologically interesting molecule.

This kind of nanoparticles can therefore be functionalized with two kinds of biologically interesting molecules.

The presence of reactive groups on the luminescent zwitterionic nanoparticles allows said nanoparticles to be easily functionalized by a natural or synthetic biologically interesting molecules through a covalent bond to said polymeric chain.

Thus, another aspect of the present invention is to provide a novel functionalized zwitterionic luminescent polymeric nanoparticle.

Said functionalized zwitterionic luminescent polymeric nanoparticle comprises at least one random copolymer comprising:
- from 0.5 to 20 mol% of repeating units having a pendant group which is negatively charged group chosen from phosphonate, sulfonate and carboxylate,
- from 3 to 30 mol%, in particular from 5 to 20 mol%, more in particular from 7 to 17 mol%, of repeating units having a pendant group which is zwitterionic group,
- higher than 0 but lower or equal to 5 mol%, of repeating units having a pendant group which is a natural or synthetic biologically interesting molecule which is covalently bound to said polymeric chain
- from 60 to 95 mol% of repeating units having a pendant group, which is a hydrophobic group.

In a particular embodiment, said functionalized zwitterionic luminescent polymeric nanoparticles of the present invention can be represented by the formula III below: 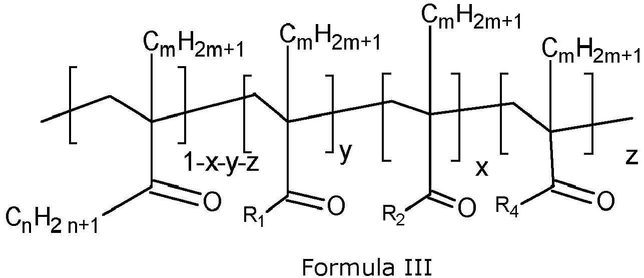
- Wherein m is 0 or 1,
- n is an integer chosen from 1 to 6
- R₁ is a zwitterionic group,
- R₂ is a negatively charged group,
- R₄ is a reactive group conjugated with a natural or synthetic biologically interesting molecule,
- x is in the range from 0.5 to 20 mol%,
- y is in the range from 3 to 30 mol%, in particular from 5 to 20 mol%, more particularly from 7 to 17 mol%,
- z is in the range higher than 0 but lower or equal to 5 mol%.

In a particular embodiment, said functionalized zwitterionic luminescent polymeric nanoparticles of the present invention can be represented by the formula IV below: 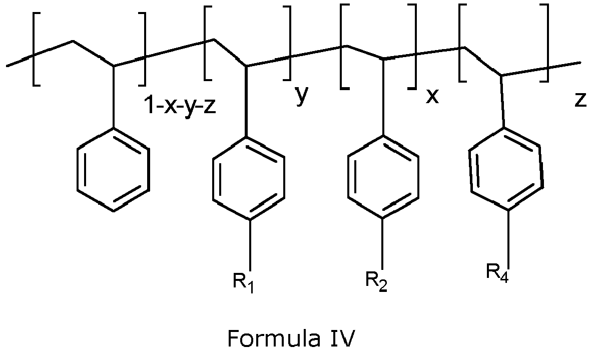
- R₁ is a zwitterionic group,
- R₂ is a negatively charged group,
- R₄ is a reactive group conjugated with a natural or synthetic biologically interesting molecule,
- x is in the range from 0.5 to 20 mol%,
- y is in the range from 3 to 30 mol%, in particular from 5 to 20 mol%, more particularly from 7 to 17 mol%,
- z is in the range higher than 0 but lower or equal to 5 mol%.

Said natural or synthetic biologically interesting molecule is chosen from an antibody, a fragment of antibody, a peptide, an aptamer, an oligonucleotide, a toxin or a chemical drug.

The presence of such natural or synthetic biologically interesting molecule on a nanoparticle confers to said nanoparticle the ability to detect a corresponding biomolecule.

According to the target biomolecule to be detected, a luminescent zwitterionic nanoparticle bearing reactive groups can be functionalized by a corresponding natural or synthetic biologically interesting molecule. For example, in order to detect the expression of a particular protein, the luminescent zwitterionic nanoparticles can be functionalized by an antibody directed to said protein.

These functionalized luminescent zwitterionic nanoparticles of the invention combining small size, ultrahigh brightness, low protein adsorption with target specificity are particularly useful for the application in *in vitro* or *in vivo* disease diagnosis, therapeutic treatment or in biological research.

The functionalized nanoparticles of the invention can be used for example as a biosensor for detecting target biomolecules, such as for detecting a particular protein, antibody or peptide.

Thanks to its high brightness, the functionalized luminescent zwitterionic nanoparticles of the invention are more sensitive, easier to use than ELISA test.

Particularly, these functionalized nanoparticles can be used as a contrast agent or medical imaging agent that can be used in *in vivo* detection, tracking of target molecules or cells for *in vivo* medical diagnosis, or as a diagnostic agent that can be used in *in vitro* detection or identification of a biomolecule or a cell expressing said biomolecule.

The present invention concerns also a method for *in vitro* or *in vivo* detection or tracking of a target biomolecule by means of a functionalized luminescent zwitterionic polymeric nanoparticle as described above.

Example of said target biological molecule can be, but not limited to, a protein, an antibody, a DNA, a RNA, a siRNA, a microRNA, a toxin.

Thanks to its ultrahigh brightness, the nanoparticle of the present invention can be used in single-molecule tracking.

According to a particular embodiment, the invention concerns a method for *in vitro* detection or tracking of a target biological molecule, in particular an antigen, in a sample.

Said sample can be a biological sample obtained from biological fluid, from an *in vitro* cell culture or from a tissue, from plants, from microorganisms, a solution containing biological molecules, an environmental sample, a food sample, a pharmaceutical sample.

By "biological fluid" is meant a liquid contained, excreted or secreted from a living animal or plant, for example: blood, different fraction of blood, lymph, bile, saliva, exudates. In a preferred embodiment of the present invention, the biological fluid is a human or animal origin fluid chosen from serum, inactivated serum, plasma, or blood.

By "tissue" is meant to a human, animal or vegetal tissue. In a particular embodiment of the invention, the sample of a tissue is a sample obtained by biopsy or during surgical operation. In a more particular embodiment, the tissue is a tumoral tissue obtained by biopsy or during surgical operation from a patient suffering from a cancer, or suspected to develop a cancer.

By "environmental sample" is meant to a sample collected from an environment, such as soil, sludge, or waste water.

For the detection, the functionalized luminescent zwitterionic nanoparticles of the invention can be suspended in solution or immobilized on surfaces of microplates.

The present invention concerns also a pharmaceutical composition comprising the functionalized luminescent zwitterionic nanoparticles of the invention for use as a contrast agent, a diagnostic agent or as medical imaging agent.

Said composition can be used as an *in vivo* diagnostic agent.

The random copolymer contained in luminescent zwitterionic hydrophobic polymeric nanoparticles as described before can be synthesized through free radical polymerization, controlled radical polymerization, condensation, addition ring opening polymerization.

The luminescent zwitterionic hydrophobic polymeric nanoparticles can be obtained by the method of nanoprecipitation.

The present invention provides also a method for preparing a functionalized fluorescent zwitterionic polymeric nanoparticle as described before, said method comprising:
- nanoprecipitation of at least one random copolymer comprising:
   - from 0.5 to 20 mol% of repeating units having a pendant group which is negatively charged group chosen from phosphonate, sulfonate and carboxylate,
   - from 3 to 30 mol%, in particular from 5 to 20 mol%, more in particular from 7 to 17 mol%, of repeating units having a pendant group which is zwitterionic group,
   - higher than 0 but lower or equal to 5 mol% of repeating units having a pendant group which is a natural or synthetic biologically interesting molecule which is covalently bound to said polymeric chain.

Another method for preparing a functionalized luminescent zwitterionic polymeric nanoparticle as described before comprises:
- the nanoprecipitation at least one random copolymer comprising:
   - from 0.5 to 20 mol% of repeating units having a pendant group which is negatively charged group chosen from phosphonate, sulfonate and carboxylate,
   - from 3 to 30 mol%, in particular from 5 to 20 mol%, more in particular from 7 to 17 mol%, of repeating units having a pendant group which is zwitterionic group,
   - higher than 0 but lower or equal to 5 mol% of repeating units having a pendant group which is a reactive group suitable to be functionalized by a natural or synthetic biologically interesting molecule,
- reaction of a luminescent zwitterionic polymeric nanoparticle obtained after nanoprecipitation with a natural or synthetic biologically interesting molecule.

The present invention is exposed more in detail in the following figures and examples.

### Figures:

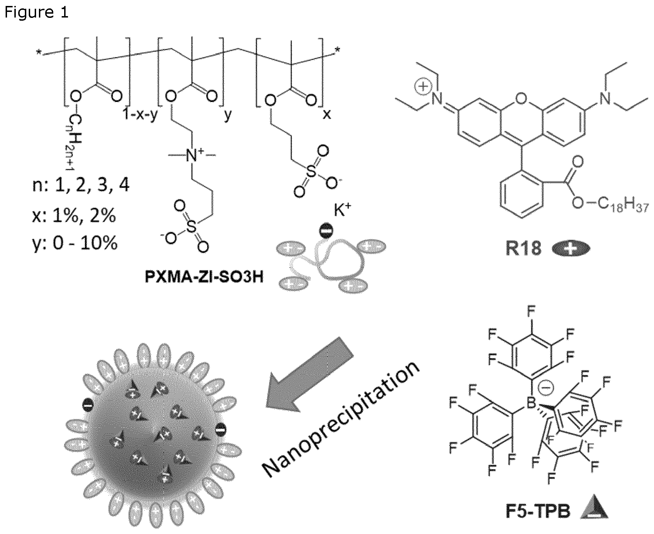

Figure 1 illustrates a simplified chemical structure of methacrylate-based copolymers bearing sulfonate and zwitterionic (ZI) group, the dye (R18) and its counterion (F5-TPB) used for preparation of zwitterionic luminescent dye-loaded NPs through nanoprecipitation. It is to be noted that the hydrophobic groups, the zwitterionic groups and sulfonate groups are randomly arranged on the backbone polymeric chain.

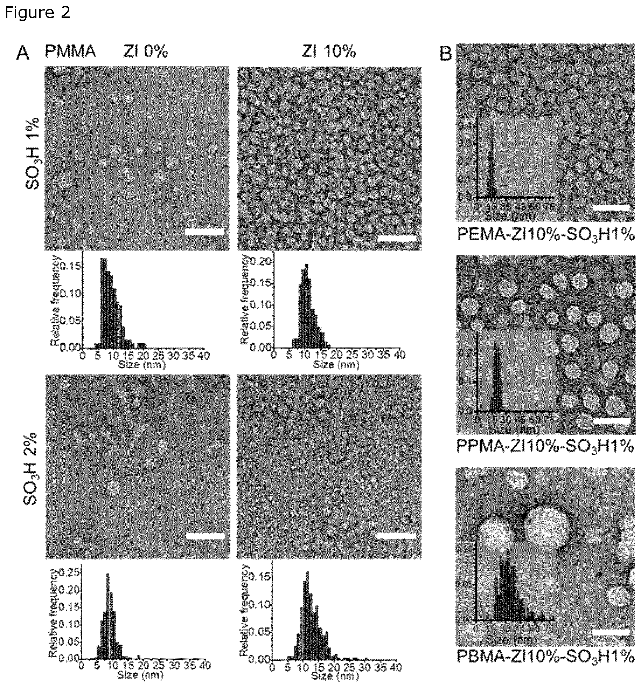

Figure 2 (A) shows TEM analysis of the sizes of zwitterionic sulfonate nanoparticles (PMMA-ZI 10%-SO₃H 1% and 2%), and non zwitterionic sulfonate nanoparticles (PMMA-SO₃H 1%, PMMA-SO₃H 2%). Nanoparticles loaded with 10 wt% R18/F5-TPB were prepared by nanoprecipitation; and Figure 2(B) shows TEM analysis of the sizes of different methacrylate polymers with 10% zwitterionic and 1% SO₃H groups. Below each image size distributions of corresponding nanoparticles are given. Scale bars correspond to 50 nm. At least 100 nanoparticles were analyzed per condition.

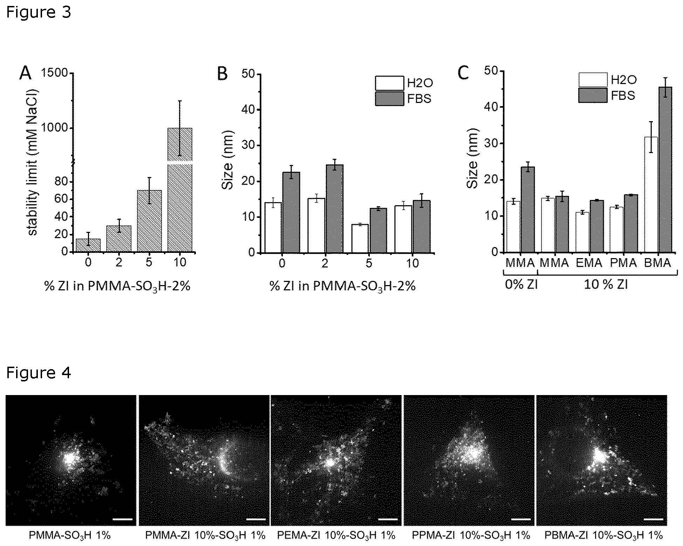

Figure 3 shows stability of NPs and their interactions with proteins as determined using FCS: (A) Influence of the ZI density on the maximum salt concentration in which the particles remained stable. (B) Size of PMMA-SO₃H 2% NPs with different ZI percentage and (C) 1% sulfonate NPs made from different methacrylate monomers with or without ZI groups in the presence of 10% fetal bovine serum (FBS). All NPs were loaded with 1% R18/F5-TPB. Given are mean values from three independent measurements. The error bars correspond to s.e.m.
Figure 4 shows representative epi-fluorescence micrographs of HeLa cells microinjected with different types of NPs loaded with 10 wt% R18/F5-TPB. Maximum projections over 60 s are shown. Scale bars correspond to 10 µm.

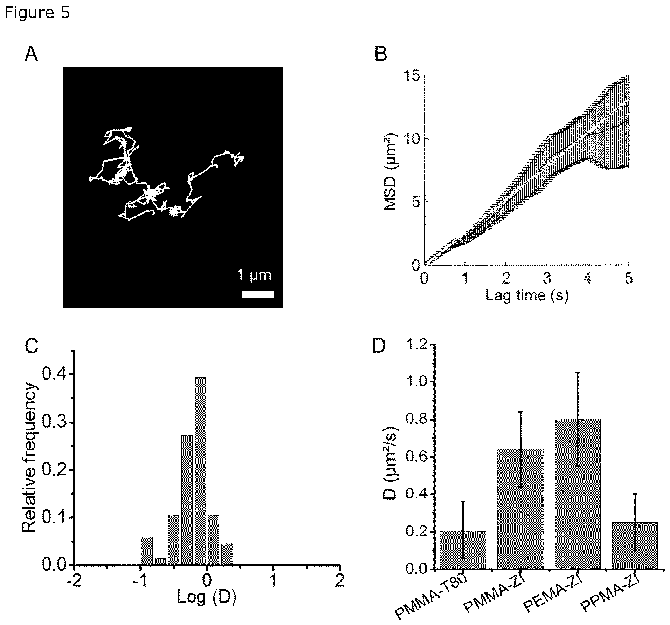

Figure 5 shows single particle tracking: (A) Trajectory of a PEMA-ZI 10%-SO₃H 1% NP loaded with 20 wt% R18/F5-TPB in the cytoplasm over 30 s. (B) Mean square displacement (MSD) of PEMA-ZI 10%-SO₃H 1% NPs. The black curve corresponds to the mean MSD curves and the straight gray line is the fitted plot. The error bars correspond to s.e.m. (C) Diffusion coefficient distribution of PEMA-ZI 10%-SO₃H 1% NPs. (D) Mean diffusion coefficients of different polymer NPs. The error bars give FWHM. At least 50 trajectories were analyzed per sample.

### Examples

### I. Materials and Methods

### Materials

Methyl methacrylate (99%, M55909), methacrylic acid (99%, 155721), 3-sulfopropyl methacrylate potassium salt (99%, 251658) and 2-(N-3-Sulfopropyl-N,N-dimethyl ammonium)ethyl methacrylate (99%, 537284) were purchased from Sigma-Aldrich. Dimethylsulfoxide (DMSO, analytical grade) was obtained from Fisher-Scientific. Milli-Q water (Millipore), acetonitrile (≥ 99.9%, Sigma-Aldrich) and methanol (≥ 99.9%, Carlo Erba reagents) were used for preparation of nanoparticles. dichloromethane (≥99.8%) from CarloErba and methanol (HPLC grade) from VWR. 6-carboxy- tetramethylrhodamine (TMR from Sigma-Aldrich), phosphate buffered saline (PBS, Fisher Scientific), fetal bovine serum (FBS, Lonza) were used for stability study. Monomers were purified using column chromatography or re-crystallization. Azobis isobutyronitrile (Aldrich, ≥ 98%) was recrystallized twice from ethanol. The other compounds were used as received. R18/F5-TPB was synthesized from rhodamine B octadecyl ester perchlorate (Aldrich, >98.0%) and lithium tetrakis(pentafluorophenyl)borate ethyl etherate (AlfaAesar, 97%) through ion exchange followed by purification through column chromatography as described previously.²

### Polymer synthesis

Synthesis of polymers of invention: The different polymers were synthesized through free radical polymerization. The different monomers were all dissolved in degased DMSO and mixed at the desired ratio. 0.01 eq. of AIBN were added and the round bottom flask was placed in an oil bath preheated to 70 °C. Once the conversion reached 25 %, the reaction was stopped and the polymers reprecipitated twice in methanol and/or water. After drying, the polymers were characterized through NMR and, where possible, size exclusion chromatography. As an example the synthesis of PMMA-SO₃H-ZI is given:
*Poly(methyl methacrylate-co-3-sulfopropyl methacrylate-co- 2-(N-3-Sulfopropyl-N,N-dimethyl ammonium)ethyl methacrylate) (PMMA-ZI-SO₃H):* Methyl methacrylate, 2-(N-3-Sulfopropyl-N,N-dimethyl ammonium)ethyl methacrylate, and 3-sulfopropyl methacrylate potassium salt were dissolved in degassed DMSO at a concentration of 2 M (1M for 2-(N-3-Sulfopropyl-N,N-dimethyl ammonium)ethyl methacrylate with addition of a small amount of methanol). The three solutions were then mixed in a 50 mL two-neck round bottom flask equipped with a stirring bar at the desired ratio to give a total volume of 20 mL. The mixture was degassed by bubbling argon for 5 min and placed under argon atmosphere. 0.01 eq. of AIBN in DMSO (40 mg/mL) were added and the round bottom flask was placed in an oil bath preheated to 70 °C. At regular intervals samples were drawn, dissolved in DMSO-d₆ and analyzed by NMR. Once the conversion reached 25 %, the reaction was stopped by quickly cooling it to RT. The reaction mixture was then added dropwise to methanol, or, for higher percentages of the charged and zwitterionic monomers, to water. After filtration, the precipitate was redissolved in a small amount of acetonitrile (when needed with a small amount of methanol) and reprecipitated twice in methanol (water for highest percentages of ZI). The obtained polymer was dried under vacuum. ¹H NMR (400 MHz, DMSO-d₆, δ): 4.36 (br, 0.04-0.2 H), 3.96 (br. s, 0.02-0.04 H), 3.55 (m, 3.4 H), 3.13 (br, 0.12-0.6 H), 2.45 (br, covered by the solvent peak), 2.04 (br, 0.04-0.2 H), 2.00-0.30 (m, 6 H). Fraction obtained of ZI and SO₃H groups based on the peak at 4.36 and 3.97 ppm, respectively (feed: obtained): ZI 10 %: 10.2 % - SO₃H 1 %: 1.4%; ZI 10 %: 11 %- SO₃H 2 %: 2.8%. Molecular weight by GPC: 1% SO₃H: M_{w} = 51 200, M_{w} /Mn = 1.58; 1% ZI: M_{w} = 43 100, M_{w} /Mn = 1.14. For polymers with higher amounts of zwitterionic monomers no suitable solvent systems for GPC were available.

*Poly(ethyl methacrylate-co-3-sulfopropyl methacrylate-co- 2-(N-3-Sulfopropyl-N,N-dimethyl ammonium)ethyl methacrylate) (PEMA-ZI-SO₃H):* ¹H NMR (400 MHz, MeOD-, δ): 4.50 (br, 0.2 H), 4.34-3.92 (m, 2.02 H), 3.81 (br, 0.2 H), 3.70 (br, 0.2 H), 3.29 (br, partial covered by the solvent peak), 2.92 (br, 0.2 H), 2.31 (br, 0.2 H), 2.23-0.5 (m, 9 H). Fraction obtained of ZI groups based on the peak at 4.50 ppm: 10.3 %.

*Poly(propyl methacrylate-co-3-sulfopropyl methacrylate-co- 2-(N-3-Sulfopropyl-N,N-dimethyl ammonium)ethyl methacrylate) (PPMA-ZI-SO₃H):* ¹H NMR (400 MHz, MeOD, δ): 4.50 (br, 0.2 H), 4.29-3.54 (m, 2.42 H), 3.29 (br, partial covered by the solvent peak), 2.92 (br, 0.2 H), 2.31 (br, 0.2 H), 2.23-0.5 (m, 11 H).

Fraction obtained of ZI groups based on the peak at 4.50 ppm: 9.4%. *Poly(butyl methacrylate-co-3-sulfopropyl methacrylate-co- 2-(N-3-Sulfopropyl-N,N-dimethyl ammonium)ethyl methacrylate) (PBMA-ZI-SO₃H):* ¹H NMR (400 MHz, CDCl₃, δ): 4.40 (br, 0.2H), 4.30-3.55 (m, 2.42 H), 3.33 (br, 0.6 H), 2.99 (br, 0.2 H), 2.36 (br, 0.2 H), 2.28-0.4 (m, 13 H). Fraction obtained of ZI groups based on the peak at 4.40 ppm: 8.3%.

Poly(methyl methacrylate-co-3-sulfopropyl methacrylate) (PMMA-SO₃H): ¹H NMR (400 MHz, DMSO-d₆, δ): 3.97 (br. s, 0.02-0.04 H), 3.57 (s, 3 H), 2.45 (m, partial covered by the solvent peak), 2.1 - 0.5 (m, 6 H). Fraction of SO₃H groups based on the peak at 3.97 ppm (feed: obtained): SO₃H 1%: 1.1%; SO₃H 2%: 2.3%.

### Preparation of nanoparticles

Stock solutions of copolymers were prepared at a concentration of 10g.l⁻¹ in acetonitrile (20 vol.% methanol for ZI polymers). These solutions were diluted at 2g.l⁻¹ in the corresponding solvent containing 1, 10 or 20 wt% of R18/F5-TPB (relative to the polymer). This solution was quickly added to a 10-fold volume excess of water, under shaking (Thermomixer comfort, Eppendorf, 1000 rpm, at 21°C) followed by a second dilution in water.

### Characterization of nanoparticles

Absorption and emission spectra were recorded on a Cary 4000 Scan ultraviolet-visible spectrophotometer (Varian) and on a FS5 Spectrofluorometer (Edinburgh Instruments) equipped with a thermostated cell compartment, respectively. The excitation wavelength was set to 530 nm and emission was recorded from 540 to 750 nm. QYs were calculated using rhodamine 101 in ethanol as reference (QY = 0.9).

Transmission electron microscopy: 5µl of nanoparticle solution were deposited onto carbon-coated copper-rhodium electron microscopy grids following amylamine glow-discharge. They were then treated for 20 s with a 2% uranyl acetate solution for staining. The obtained grids were observed using a Philips CM120 transmission electron microscope equipped with a LaB6 filament and operating at 100kV. The acquisition of areas of interest was recorded with a Peltier cooled CCD camera (Model 794, Gatan, Pleasanton, CA). Images were analyzed using Fiji software.

Fluorescence correlation spectroscopy: Measurements were performed on a home-built confocal set-up using excitation at 532 nm using TMR in water as reference. The solution of NPs containing 1 wt% dyes were diluted 2 times before depositing 200 µl on 96-well optical-bottom plates for measurements. NPs stability in presence of salts and proteins were investigated by adding drop-by-drop 50 vol. % of 10-fold PBS (10x), FBS or water to the solutions of NPs in low-binding 1.5ml Eppendorf tubes. The data were recorded 5 min after addition and then analyzed using the PyCorrFit software.

### Cellular experiments:

HeLa cells were grown in Dulbecco's modified Eagle medium (DMEM, without phenolred, Gibco-Invitrogen), supplemented with 10% fetal bovine serum (FBS, Lonza), L-glutamine, and 1% antibiotic solution (penicillin-streptomycin, Gibco-Invitrogen) at 37° C in humidified atmosphere containing 5% CO₂. Cells were seeded onto a round microscope cover glasses (diameter 18 mm) deposited in 6 well plates at a density of 125×10³ cells/well 24h before the microinjection.

Microinjection of NPs and cellular imaging: For microinjection experiments, subconfluent HeLa cells plated on glass coverslips were mounted in a Ludin Chamber (Life Imaging Services, Basel, Switzerland). The cells were then placed on a Leica DMIRE 2 microscope (37°C, 5% CO₂, 100x objective, sCMOS camera, Xenon lamp) and solutions of the different nanoparticles at particle concentrations of 0.5 to 2 nM were microinjected into the perinuclear region of the cells, using a Femtojet/InjectMan NI2 microinjector (Eppendorf). Images sequences were then acquired either on the same setup or on an iMIC microscope (Till Photonics) equipped with a Mutli-LED Spectra X (Lumencor), an Olympus 60x TIRFM (1.45 NA) objective, and an Flash 4 V2+ camera (Hamamatsu) after transfer of the samples.

Live cells were maintained at 37°C in a 5% CO₂ humidified atmosphere using an environmental control system (Life Imaging Services). Time-lapse movies were recorded over 60 s with a frame rate of 50 ms and binning 2. They were then analyzed using the ImageJ (National Institutes of Health, USA). For single particle tracking, time-lapse movies were recorded over 30 s with a frame rate of 43 ms for the Imic microscope or 50 ms for the Leica DMIRE 2 microscope and a binning 1 in order to increase the resolution. With Fiji software, the trajectories were recovered from TrackMate plugin. Then MSD curves were plotted and slopes extracted from the MSD curves with a MATLAB script for particle tracking analysis.

### II. Experimental Results

### Influence of negative charged groups and zwitterionic groups on the particle size

Methacrylate based copolymers bearing sulfonate and zwitterionic (ZI) groups were synthetized according to the method described above in "Materials and Methods". In the following text the polymers are noted PXMA-ZI-x%-SO3H-y%, where XMA stands for the corresponding major monomer (MMA for methyl methacrylate, EMA for ethyl methacrylate, PMA for propyl methacrylate, BMA for butyl methacrylate), x and y correspond to the molar percentage of the zwitterionic and sulfonate groups. By the way, polymers bearing only sulfonate but no zwitterionic groups were also prepared as control.

These polymers were then used to assemble dye-loaded polymer NPs through nanoprecipitation. For this, solutions of the polymers in acetonitrile (with a small amount of methanol) containing different amounts of the salt of a rhodamine B derivative (R18) with a perfluorinated borate (F5-TPB) were added quickly to a large excess of water. The size of the formed NPs was analyzed through transmission electron microscopy (TEM, Figure 2, Table 1).

In the case of polymers bearing only sulfonate but no zwitterionic groups, very small particles were observed: PMMA-SO₃H 1% yielded NPs of about 13 nm and the particle size decreased to about 9 nm for 2% sulfonate. The introduction of 10% ZI on the polymer chains had only a minor influence on the particle size of PMMA-ZI-SO₃H based NPs. They reached 11 and 9 nm of diameter, respectively for 1% and 2% sulfonate NPs. The presence of the ZI groups hence did not affect the process of particle formation and the obtained "ZI shell" is thin enough for not influencing the size of NPs. Increasing the hydrophobicity of the alkyl methacrylate monomers on the other hand led to increasing particle size (Figure 2, Table 1) in the order PMMA-ZI 10%-SO₃H 1% < PEMA-ZI 10%-SO₃H 1% < PPMA-ZI 10%-SO₃H 1% < PBMA-ZI 10%-SO₃H 1%, from 11 to 35 nm.

**Table 1. Sizes of NPs made from different polymers as obtained from transmission electron microscopy and fluorescence correlation spectroscopy. Errors correspond to width of the distribution at half maximum for TEM, and variation over 3 measurements for FCS.**

| Polymer | | | Size (nm) | |
|---|---|---|---|---|
| Main monomer | SO₃H | ZI | TEM¹⁾ | FCS²⁾ |
| MMA | 1 mol% | - | 13 ± 3 | 14 ± 1 |
| MMA | 1 mol% | 10 mol% | 11 ± 3 | 15 ± 1 |
| MMA | 2 mol% | - | 9 ± 3 | 14 ± 1 |
| MMA | 2 mol% | 10 mol% | 9 ± 2 | 13 ± 1 |
| EMA | 1 mol% | 10 mol% | 14 ± 3 | 11 ± 1 |
| PMA | 1 mol% | 10 mol% | 22 ± 4 | 13 ± 1 |
| BMA | 1 mol% | 10 mol% | 35 ± 7 | 32 ± 4 |

| | | | | |
|---|---|---|---|---|
| 1) NPs prepared with 10 wt% R18/F5-TPB. 2) NPs prepared with 1 wt% R18/F5-TPB. | | | | |

The nanoparticles were made fluorescent through the encapsulation of high amounts of dyes (between 1 and 20 wt% relative to the polymer). Here, the cationic rhodamine R18 was associated to the large and very hydrophobic counterion F5-TPB as this association avoids aggregation-caused quenching and leads to a very hydrophobic dye counterion pair. Dialysis of the dye-loaded NPs over 48 h showed a release of less than 5% of the used dye, indicating that this approach yielded very efficient encapsulation of the dye salt in the particles, in agreement with previous results showing very efficient encapsulation of dyes with F5-TPB counterion.¹⁵ Furthermore, the quantum yield of the particles remained > 30% for a 10 wt% loading for all zwitterion bearing polymers, thus ensuring excellent particle brightness.

### Stability of zwitterionic polymeric nanoparticles

The stability of the resulting particles in biological media, and in particular the potential of the zwitterionic groups to improve this, was investigated by fluorescence correlation spectroscopy (FCS). The percentage of ZI groups in the polymers was varied from 0 to 10% in order to modify the density of ZI on the particle. The particle sizes of NPs obtained from FCS data were in very good agreement with those obtained from TEM (Figure 3, Table 1. The smaller size is associated with the lower amount of dye salt used in FCS experiments.). In a first step, the stability of NPs was evaluated through addition of NaCl solutions with increasing concentrations (Figure 3A). The stability limit was defined as the last NaCl concentration for which no aggregation of NPs was observed. Particles made from polymers bearing no ZI groups already started to aggregate (and precipitate) as soon as a small amount of salt was added (Figure 3A). Addition of ZI groups led to an increase in the particle stability with increasing amount of ZI groups. At 10% of zwitterionic groups the NPs did not show any change in size and no signs of precipitation up to 1 M NaCl.

The influence of the zwitterionic groups on interactions of the NPs with proteins and other biomolecules was then tested by adding fetal bovine serum (FBS), a complex mixture of salts and biomolecules containing notably numerous proteins (Figure 3B). In the case of particles without ZI groups a size increase of about 10 nm was observed, which corresponds to the adsorption of at least a monolayer of proteins and thus the formation of a "hard" protein corona (Figure 3B). Starting from 5% of ZI groups the size increase of the NPs upon interaction with proteins was significantly reduced. At 10% of ZI groups no significant increase in particle size was observed, indicating that the surfaces of these particles resisted protein adsorption (Figure 3C).

### In vitro comportment of zwitterionic polymeric nanoparticles

These NPs were then directly microinjected in the perinuclear region of the cytosol of living HeLa cells and their behavior was monitored using epifluorescence microscopy (Figure 4). In all cases, diffuse staining of cytosolic structures was virtually absent. This confirms the efficient and stable encapsulation of the dyes within the NPs and the absence of dye leaching. Maximum projections of the fluorescence intensities over 60s gave a general idea of the overall distribution of the particles throughout the cytosol: In the absence of ZI groups, most of the particles remained stuck at the injection point and the few particles in the cytosol had a low mobility, indicating strong interactions of the particles with the cellular constituents. On the other hand, particles bearing 10% ZI groups distributed, in general, well throughout the cytosol. PMMA-based ZI-bearing NPs showed a distribution all over the cytosol and a high mobility, even though there is sometime a "projection" on the nucleus close to the injection point. PEMA-ZI particles showed a homogeneous distribution and high mobility for practically all NPs. This was also observed for PPMA-ZI particles, though here a certain part of the particles remained close to the injection point. In the case of PBMA-based particles, they were more localized at the injection point than particles based on other tested polymers. These results confirmed that 10% ZI groups are sufficient to strongly reduce interactions of the particles with cellular constituents and enable spreading throughout the cytosol. However, these results also confirm that the particle size also has a major influence on intracellular particle diffusion, and only particles below a critical core size of around 23 nm can spread throughout the cytosol due to steric hindrance by cellular structures.⁶ Here, the PBMA particles had sizes clearly above this threshold, leading to their immobilization. Part of the PPMA particles were also above this threshold, resulting in immobilization of part of the particles.

### Single particle tracking

The high brightness of the particles of the invention enabled monitoring the mobility and diffusion behavior of the NPs at the single particle level and so to better understand the influence of the ZI groups and the type of polymer used on the intracellular behavior of our NPs. An example of a cytoplasmic trajectory of a PEMA-based ZI-bearing NP is represented in Figure 5A. Plotting the mean square displacement (MSD) *vs* lag time for these NPs showed a linear increase up to about 10 µm² with an exponent α of 1 (Figure 5B). This indicates normal or Brownian diffusion of these particles in the cytosol, which is described, in two dimensions, as MSD = 4DΔt^{α} with D, the diffusion coefficient and α = 1.¹⁶ The diffusion coefficients of individual NPs were then extracted from the corresponding MSD curves. Figure 5C shows that for PEMA-ZI NPs the distribution of the diffusion coefficients is centered around 1 µm².s⁻¹, with a mean D of 0.80 µm².s⁻¹, in good agreement with values obtained for QDs of similar size.⁴ The distribution shows only a small fraction of NPs with diffusion coefficients below 0.2 µm².s⁻¹. PMMA-ZI NPs had a similar distribution of diffusion coefficients with a slightly lower mean of 0.65 µm².s⁻¹ (Figure 5D), in agreement with the slightly larger hydrodynamic size. PPMA-ZI NPs, on the other hand, showed a clearly lower mean diffusion coefficient of 0.25 µm².s⁻¹. This is probably due to their larger size leading to a restricted cytoplasmic diffusion, as already indicated by their stronger clustering around the injection point. As PMMA-SO₃H 1% NPs without ZI groups remained clustered around the injection point, and hence it was not possible to characterize their diffusion behavior. However, we could show earlier that simple adsorption of Tween 80 (T80), a PEGylated surfactant, on such nanoparticles strongly reduces their interaction with proteins and permits their diffusion in the cytosol.^{5,15} Interestingly, the mean diffusion coefficient of these NPs was with 0.2 µm².s⁻¹ three to four fold lower than those of the PMMA and PEMA ZI NPs, even though their core sizes were very close (Figure 2). One reason for this is certainly the fact that the addition of the PEGylated surfactant increases the NP size by > 5 nm. The large difference in diffusion coefficients further indicates that the ZI groups are more effective in reducing non-specific interactions with intracellular biomolecules and structures than the adsorbed PEG shell.
(1) Li, K.; Qin, W.; Ding, D.; Tomczak, N.; Geng, J.; Liu, R.; Liu, J.; Zhang, X.; Liu, H.; Liu, B.; et al. Photostable Fluorescent Organic Dots with Aggregation-Induced Emission (AIE Dots) for Noninvasive Long-Term Cell Tracing. Sci. Rep. 2013, 3. https://doi.org/10.1038/srep01150.
(2) Reisch, A.; Didier, P.; Richert, L.; Oncul, S.; Arntz, Y.; Mély, Y.; Klymchenko, A. S. Collective Fluorescence Switching of Counterion-Assembled Dyes in Polymer Nanoparticles. Nat. Commun. 2014, 5. https://doi.org/10.1038/ncomms5089.
(3) Sahl, S. J.; Hell, S. W.; Jakobs, S. Fluorescence Nanoscopy in Cell Biology. Nat. Rev. Mol. Cell Biol. 2017, 18 (11), 685-701. https://doi.org/10.1038/nrm.2017.71.
(4) Etoc, F.; Balloul, E.; Vicario, C.; Normanno, D.; Liße, D.; Sittner, A.; Piehler, J.; Dahan, M.; Coppey, M. Non-Specific Interactions Govern Cytosolic Diffusion of Nanosized Objects in Mammalian Cells. Nat. Mater. 2018, 17(8), 740-746. https://doi.org/10.1038/s41563-018-0120-7.
(5) Reisch, A.; Heimburger, D.; Ernst, P.; Runser, A.; Didier, P.; Dujardin, D.; Klymchenko, A. S. Protein-Sized Dye-Loaded Polymer Nanoparticles for Free Particle Diffusion in Cytosol. Adv. Funct. Mater. 2018, 28 (48), 1805157. https://doi.org/10.1002/adfm.201805157.
(6) Luby-Phelps, K. Cytoarchitecture and Physical Properties of Cytoplasm: Volume, Viscosity, Diffusion, Intracellular Surface Area. Int. Rev. Cytol. 2000, 192, 189-221.
(7) Walkey, C. D.; Chan, W. C. W. Understanding and Controlling the Interaction of Nanomaterials with Proteins in a Physiological Environment. Chem. Soc. Rev. 2012, 41 (7), 2780-2799. https://doi.org/10.1039/ClCS15233E.
(8) Monopoli, M. P.; Åberg, C.; Salvati, A.; Dawson, K. A. Biomolecular Coronas Provide the Biological Identity of Nanosized Materials. Nat. Nanotechnol. 2012, 7 (12), 779-786. https://doi.org/10.1038/nnano.2012.207.
(9) Jokerst, J. V.; Lobovkina, T.; Zare, R. N.; Gambhir, S. S. Nanoparticle PEGylation for Imaging and Therapy. Nanomed. 2011, 6 (4), 715-728. https://doi.org/10.2217/nnm.11.19.
(10) Rabanel, J.-M.; Hildgen, P.; Banquy, X. Assessment of PEG on Polymeric Particles Surface, a Key Step in Drug Carrier Translation. J. Controlled Release 2014, 185, 71-87. https://doi.org/10.1016/j.jconrel.2014.04.017.
(11) Estephan, Z. G.; Schlenoff, P. S.; Schlenoff, J. B. Zwitteration As an Alternative to PEGylation. Langmuir 2011, 27 (11), 6794-6800. https://doi.org/10.1021/la200227b.
(12) Garcia, K. P.; Zarschler, K.; Barbaro, L.; Barreto, J. A.; O'Malley, W.; Spiccia, L.; Stephan, H.; Graham, B. Zwitterionic-Coated "Stealth" Nanoparticles for Biomedical Applications: Recent Advances in Countering Biomolecular Corona Formation and Uptake by the Mononuclear Phagocyte System. Small 2014, 10 (13), 2516-2529. https://doi.org/10.1002/smll.201303540.
(13) Muro, E.; Pons, T.; Lequeux, N.; Fragola, A.; Sanson, N.; Lenkei, Z.; Dubertret, B. Small and Stable Sulfobetaine Zwitterionic Quantum Dots for Functional Live-Cell Imaging. J. Am. Chem. Soc. 2010, 132 (13), 4556-4557. https://doi.org/10.1021/ja1005493.
(14) Rouhana, L. L.; Jaber, J. A.; Schlenoff, J. B. Aggregation-Resistant WaterSoluble Gold Nanoparticles. Langmuir 2007, 23 (26), 12799-12801. https://doi.org/10.1021/la702151q.
(15) Reisch, A.; Runser, A.; Arntz, Y.; Mély, Y.; Klymchenko, A. S. Charge-Controlled Nanoprecipitation as a Modular Approach to Ultrasmall Polymer Nanocarriers: Making Bright and Stable Nanoparticles. ACS Nano 2015, 9 (5), 5104-5116. https://doi.org/10.1021/acsnano.5b00214.
(16) Ruthardt, N.; Lamb, D. C.; Bräuchle, C. Single-Particle Tracking as a Quantitative Microscopy-Based Approach to Unravel Cell Entry Mechanisms of Viruses and Pharmaceutical Nanoparticles. Mol. Ther. 2011, 19 (7), 1199-1211. https://doi.org/10.1038/mt.2011.102.

## Claims

1. A zwitterionic luminescent polymeric nanoparticle comprising at least one random copolymer, said random copolymer comprising :
- from 0.5 to 20 mol% of repeating units having a pendant group, which is a negatively charged group chosen from phosphonate, sulfonate and carboxylate,
- from 3 to 30 mol%, in particular from 5 to 20 mol%, more particularly from 7 to 17 mol%, of repeating units having a pendant group, which is a zwitterionic group,
- from 0 to 5 mol% of repeating units having a pendant group, which is a reactive group which is suitable to be functionalized by a natural or synthetic biologically interesting molecule,
- from 60 to 95 mol% of repeating units having a pendant group, which is a hydrophobic group.

2. The zwitterionic luminescent polymeric nanoparticle according to claim 1, wherein said random copolymer is chosen from a (C1-C6)alkyl methacrylate based polymer ,an (C1-C6)alkyl acrylate based polymer, an acrylamide based polymer, a polyester based polymer, a polyamide (polypetide) based polymer, a styrene based polymer and copolymers thereof.

3. The zwitterionic luminescent polymeric nanoparticle according to claim 1 or 2, wherein the zwitterionic group is chosen from ammoniophosphates, ammoniophosphonates, ammoniophosphinates, ammoniosulfonates, ammoniosulfates, ammoniocarboxylates, ammoniosulfonamides, ammoni-sulfon-imides, guanidiniocarboxylates, pyridiniocarboxylates, pyridiniosulfonates, ammonio(alkoxy)dicyanoethenolates, ammonioboronates, sulfoniocarboxylates, phophoniosulfonates, phosphoniocarboxylates.

4. The zwitterionic luminescent polymeric nanoparticle according to any one of claims 1 to 3, wherein said random copolymer is a (C1-C6)alkyl methacrylate based polymer, and comprises :
- from 0.5 to 20 mol% of repeating units having a pendant group which is a negatively charged group chosen from phosphonate, sulfonate and carboxylate,
- from 3 to 30 mol%, in particular from 5 to 20 mol%, more in particular from 7 to 17mol%, of repeating units having a pendant group which is a zwitterionic group,
- from 0 to 5 mol% of repeating units having a pendant group which is a reactive group which is suitable to be functionalized by a natural or synthetic biologically interesting molecule,
- from 60 to 95 mol% of repeating units having a pendant group which is a (C1-C6)alkyl.

5. The zwitterionic luminescent polymeric nanoparticle according to any one of claims 1 to 4, wherein the nanoparticle comprises a fluorescent dye optionally with its counter-ion, said dye and said counterions being encapsulated inside the nanoparticle.

6. The zwitterionic luminescent polymeric nanoparticle according to any one of claims 1 to 5, wherein the fluorescent dye is chosen from a rhodamine derivative or a cyanine derivative.

7. The zwitterionic luminescent polymeric nanoparticle according to any one of claims 1 to 6, wherein the nanoparticle has a diameter of from 5 nm to 200 nm, particularly from 5 nm to 150 nm, more particularly from 5 nm to 100 nm, still more particularly from 5 nm to 50 nm, still more particularly from 7 to 30 nm, still more particularly from 7 to 20 nm.

8. A functionalized zwitterionic luminescent polymeric nanoparticle comprising at least one random copolymer, said random copolymer comprising :
- from 0.5 to 20 mol% of repeating units having a pendant group, which is a negatively charged group chosen from phosphonate, sulfonate and carboxylate,
- from 3 to 30 mol%, in particular from 5 to 20 mol%, more in particular from 7 to 17 mol% of repeating units having a pendant group, which is a zwitterionic group,
- higher than 0 but lower or equal to 5 mol% of repeating units having a pendant group which is a natural or synthetic biologically interesting molecule, which is covalently bound to said polymeric chain,
- from 60 to 95 mol% of repeating units having a pendant group, which is a hydrophobic group.

9. The functionalized zwitterionic luminescent polymeric nanoparticle according to claim 8, wherein said natural or synthetic biologically interesting molecule is chosen from an antibody, a fragment of antibody, a peptide, an aptamer, an oligonucleotide, a toxin or a chemical drug.

10. The functionalized zwitterionic luminescent polymeric nanoparticle according to claim 8 or 9, for its use as a contrast agent, diagnostic agent or as medical imaging agent.

11. Method for *in vitro* or *in vivo* detection or tracking of a target biological molecule by means of a zwitterionic functionalized fluorescent dye-loaded polymeric nanoparticle according to claim 8 or 9.

12. Method according to claim 11, for *in vitro* detection or tracking of a target biological molecule, in particular an antigen, in a sample.
